(19) Europäisches Patentamt
European Patent Office
Office européen des brevets

(11) Publication number: 0 326 987 A2

(12) # EUROPEAN PATENT APPLICATION

(21) Application number: 89101535.6

(22) Date of filing: 30.01.89

(51) Int. Cl.[4]: A61K 31/35

A request for correction of the description has been filed pursuant to Rule 88 EPC. A decision on the request will be taken during the proceedings before the Examining Division (Guidelines for Examination in the EPO, A-V, 2.2).

(30) Priority: 05.02.88 US 152501
19.10.88 US 259662

(43) Date of publication of application:
09.08.89 Bulletin 89/32

(84) Designated Contracting States:
BE CH DE FR GB GR IT LI NL SE

(71) Applicant: EASTMAN KODAK COMPANY (a New Jersey corporation)
343 State Street
Rochester New York 14650(US)

(72) Inventor: Mickle, Donald Alexander Gordon
7 McGillivray Avenue
Toronto Ontario M5M 2X9(CA)
Inventor: Wu, Tai-Wing
333 Glengrove Avenue West
Toronto Ontario M5N 1W4(CA)

(74) Representative: Baillie, Iain Cameron et al
c/o Ladas & Parry Isartorplatz 5
D-8000 München 2(DE)

(54) **Compositions and methods of protecting mammalian tissue from reperfusion injury.**

(57) Administration of a chroman, 6-hydroxy-2,5,7,8-tetramethylchroman-2-carboxylic acid, or a mixture of chroman and ascorbic acid, prior to or simultaneously with resuming normal blood supply to mammalian tissue following ischemia provides substantial protection from tissue damage that otherwise is observed upon reperfusion.

EP 0 326 987 A2

Xerox Copy Centre

This invention relates to the prevention or reduction of tissue damage that generally has been observed to occur upon reperfusion following ischemia caused, e.g., by blood clots or organic repair or transplant surgery.

A number of causes and mechanisms have been suggested for the damage that occurs to tissue after ischemia and reperfusion. While it is likely that a variety of causes and mechanisms contribute to the damage, a popular current theory that is supported by experimental evidence involves the generation of free radicals upon reperfusion. See J.L. Marx, "Oxygen Free Radicals linked to Many Diseases, "Science, Research News, 30 January 1987, pp. 529-53L.

With respect to myocardial tissue, a recent publication, "Evidence of myocardial free radical injury during elective repair of tetralogy of Fallot, "[Del Nido et al. Circulation, 76 Supplement V, v-174, (1987)] indicates that lipid peroxidation (probably caused by an oxygen mediated free radical mechanism) occurs in pediatric patients during surgery to correct Tetralogy of Fallot. The current consensus on the role of free radicals in reperfusion injury in the heart is discussed in a recent review article entitled "Free radicals and myocardial ischemia and reperfusion injury, "(Simpson and Lucchesi J Lab Clin Med, July, 1987) which lists 146 references.

Hansson et al. in an article entitled "Kidney protection by pretreatment with free radical scavengers and allopurinol: renal function at recirculation after warm ischemia in rabbits" [Clin Sci 71, 245-251 (1986)] concluded that renal ischemia results in peroxidative injury. Similarly, Bilkenko etal, [Byull. Eksp. Biol. Med. 96, 9 (1983)] reported that the antiischemic effect of 2,6-di-t-butyl-4-methyl phenol in kidney reperfusion was related to its ability to inhibit the process of lipid peroxidation.

The peroxidation of polyunsaturated fatty acids due to free radical formation has received attention as a primary factor in the irreversible cellular damage caused by cerebral ischemia and subsequent recirculation, [Flamm et al. Stroke 9, 445-447 (1978)] and in post-traumatic spinal chord ischemia [Hall and Wolf, J. Neurosurg. 64, 951-961 (1986)].

The role of free radical scavenging and cell membrane protection against lipid peroxidation by Vitamin E, Vitamin C or other antioxidants in vitro during reperfusion of ischemic skin flaps has been described by Hayden et al. [Laryngoscope 97, 1176-1179(1987)]

Cellular damage caused by hepatic ischemia and reperfusion has been explained on the basis of free radical peroxidation [Marubayashi et al. Surgery 99, 184-192 (1986).]

Both Vitamin E (alpha-tocopherol), which is very lipophilic, and its somewhat more water soluble derivative, 6-hydroxy-2,5,7,8,-tetramethylchroman-2-carboxylic acid (also known as Trolox-C, hereinafter sometimes referred to as TX-C for convenience), are known antioxidants that have been used as food additives to retard spoilage. Mixtures of TX-C and ascorbic acid are also known as food preservatives [Cort U.S. Patent 3,903,317 (9-2-75)].

Both Vitamin E and Trolox C are chromans; their structures are shown below.

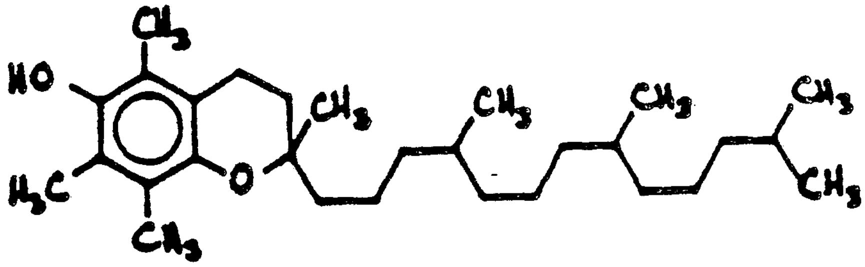

VITAMIN E

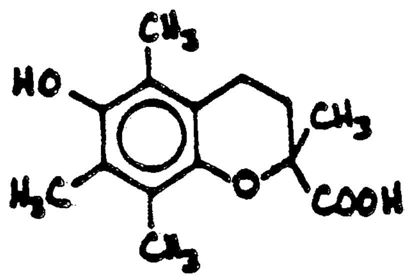

TX-C

The prophylactic use of Vitamin E to reduce the amount of tissue damage upon reperfusion of ischemic tissue in the kidney [Takenaka et al. Transplantation 32, 137-141 (1981)], in the brain [Fujimoto et al. Surg. Neurol 22, 449-454 (1984)], and in the liver [Marubayashi et al. op. cit.] has been described. In the heart, the prophylactic use of Vitamin E has been reported to be successful in some paradigms [Cavarocchi et al. J. Surg. Research 40, 519-527 (1986) and Sarrett et. al. Cardiologia 31, 539-544(1986);Biol. Abs. 84063823 (1987)] but not in others [Ferrari et al. Acta Vitaminol Enzymol 7 Suppl., 61-70 (1985)]. In the lung, Vitamin E was without effect on reperfusion injury [Bishop et al. Am. Rev. Respir. Dis. 133 part 2, A275 (1986)].

Trolox C when used in the presence of excess aspirin thymine or phenylalanine has been reported to protect alcohol dehydrogenase from radiation damage [Gee et al. Brit. J. Radiol. 58, 251-256 (1985)] It is unclear from this report whether ethylene diamine tetraacetic acid was present in the compositions tested when Trolox C was present. Some of the experiments reported used small amount of an ethylenediaminetetraacetic acid-phosphate buffer for the alcohol dehydrogenase being tested.

Trolox C dissolved in propylene glycol has also herein reported to protect rat liver from the peroxidative damage caused by halo-benzene poisoning [Casini et al. Am. J. Pathol. 118, 225-237 (1985)], although it did not protect isolated hepatocytes from herbicide poisoning [Sandy et al. Biochem. Phermacol. 35, 3095-3101 (1986)]. In this case the hepatocytes were suspended in Kreb s-Hensleit buffer to which albumin had been added. Trolox C was also reported to protect isolated perfused rabbit lungs from the pulmonary arterial pressure reaction provoked by the perfusion with a calcium ionophore; the effect was attributed to inhibition of cyclooxygenase and lipoxygenase activity by antioxidants. [Wolf and Seeger Ann. N.Y. Acad. Sci. 393, 392-410(1982)]. We are unaware of any reports of the use of Trolox C to treat or prevent reperfusion injury in any tissue.

Kato et al. [J. Med. Chem. 31, 793-798(1988)] reported that several acylated 2,3-enediol congeners of ascorbic acid were effective, when given prophylactically, in inhibiting reperfusion injury in the rat LAD model of myocardial ischemia. They further reported that both Vitamin E and ascorbic acid were without effect in this model, Woodward and Zakaria [J. Molec.Cell. Cardiol. 17, 485-493 (1985)] reported that ascorbic acid introduced after restriction and before reperfusion, reduced the incidence and duration of ventricular fibrillation in an isolated perfused rat heart model of reperfusion induced arrhythmia. Khuzhamberdiev [Bull.Exp.Biol. Med.100, 1179-1181 (1985)]suggested the combined administration of Vitamins E and C to potentiate the therapeutic effect of prophylactically administered Vitamin E in the treatment of patients with chronic ischemic heart disease.

With one exception, all reports in the literature of the use of Vitamin E to limit reperfusion injury describe administering Vitamin E prophylactically for some period before the ischemic event. Those of ordinary skill in the art are aware that the results of administration of a therapeutic agent before the insult

are in no way predictive of results of administration of the same agent in the acute stage of the injury. In the one reported case of the administration of Vitamin E after a cerebral ischemic event, the Vitamin E was a component of a complex mixture of antioxidants in a fluorocarbon vehicle and the results cannot be ascribed to any individual component [Oba et al. No Shinkei Geka(Neurol. Surg.)13, 1059-1065 (1985); Excerpta Medica 85240988].

To date, the most efficacious post ischemic treatment that has been reported for preventing reperfusion injury has been provided by superoxide dismutase(SOD). Thus, for example, Ambrosio et al. Circulation 74, 1424-1433, (1986), report that SOD perfusion (alone or together with catalase) simultaneously with reperfusion of the ischemic dog heart resulted in an infarct size of only 33.6% of the risk region as compared to 52.2% of the risk region in control dogs, a significant 36% improvement.

As reported hereinafter, we have surprisingly found that in myocardial ischemia the treatment of the present invention provides even greater protection than that reported for SOD (infarct of 8.2% of the risk region vs. 27.4% for the control, an improvement of about 80%).

Thus, we have discovered that administration of Trolox C, preferably together with ascorbic acid, preferably dissolved in saline, substantially concurrently with reperfusion of ischemic tissue, is a very effective way to prevent or minimize reperfusion injury.

More particularly, in accordance with the present invention there is provided a method for protecting a mammal from tissue injury that is associated with reperfusion following ischemia, which method comprises introducing into the blood circulation of said mammal an amount effective to inhibit reperfusion injury of the chroman compound 6-hydroxy-2,5,7,8-tetramethylchroman-2-carboxylic acid, or of a mixture of said chroman and ascorbic acid.

In another aspect, the present invention provides a composition for protecting a mammal from tissue injury associated with reperfusion after ischemia, said composition comprising an effective amount of 6-hydroxy-2,5,7,8-tetramethylchroman-2-carboxylic acid or of a mixture of said chroman and ascorbic acid in a pharmaceutically acceptable vehicle. In order to minimize the risk of oxidation of Trolox C, such compositions should contain a pharmaceutically acceptable chelating agent (such as ethylene diamine tetraacetic acid) to remove any metal ions (such as iron) that might catalize oxidation of Trolox C. For the same reason, the pharmaceutically acceptable vehicle used should be treated prior to use to eliminate as far as possible the presence of oxygen. This may be accomplished for example by prolonged purging with nitrogen. The compositions should be protein-free.

In yet another aspect, the present invention provides a process for preparing a preferred composition for carrying out the treatment of the present invention, this process comprising:

(1) bubbling nitrogen through physiological saline solution for a period of from about one to about three hours in order to reduce the oxygen content of said solution;

(2) adding from about 25 to about 50 mg of ethylenediaminetetraacetic acid (EDTA) per liter of the saline solution while continuing said nitrogen bubbling;

(3) adding approximately 4-6 gm of ascorbic acid per liter of the saline solution while continuing nitrogen bubbling;

(4) adding from about 3 to about 6 gm of TX-C per liter of saline while stirring and bubbling nitrogen;

(5) slowly (over a period of approximately 2 to 10 minutes) adding sodium hydroxide to adjust the pH of the resulting solution to about 12 so as to enhance the solubility of the TX-C;

(6) adding from about 2 to about 6 gm of ascorbic acid per liter of saline solution or sufficient hydrochloric acid so as to achieve a pH less than or equal to a value within the range of about 6.5 to about 8.0; and

(7) slowly (over a period of approximately 2 to 10 minutes) adding sodium hydroxide if and to the extent needed to adjust the pH to a value within the range of about 6.5 to about 8.0.

In a preferred composition for use in the treatment of the present invention, the TX-C or TX-C with ascorbate is provided as a liquid solution. More preferably, the solution is a physiological saline solution comprising water, sodium chloride, TX-C, ascorbic acid and ethylenediaminetetraacetic acid. Preferably, it has a pH in the range of from about 6.5 to about 8.0. More preferably, the pH is in the range of from about 7.2 to about 7.8, most preferably about 7.4. A preferred composition in accordance with the present invention comprises physiological saline solution having added thereto about 3 to 6 grams per liter of TX-C, from about 4 to about 10 grams per liter of ascorbic acid, from about 25 to about 50 mg per liter of ethylenediaminetetraacetic acid and such amounts of sodium hydroxide and hydrochloric acid as are needed to provide the desired pH. The presently particularly preferred composition for myocardial protection comprises from about 3.5 to about 4.5 grams, most preferably about 4 grams, per liter of TX-C, from about 5 to about 8 grams, most preferably about 6 grams, per liter of ascorbic acid and from about 30

to about 40 mg, most preferably about 35 mg, per liter of ethylenediaminetetraacetic acid. The presently preferred composition for liver protection comprises about 6 grams per liter of TX-C, about 6 grams per liter of ascorbic acid and about 40 mg per liter of ethylenediaminetetraacetic acid.

Other pharmaceutically acceptable vehicles may be substituted for the presently preferred vehicle described above. Thus, for example, physiological saline could be replaced by from about 2 to about 5% (wt./vol.) dextrose solution, or Ringer's lactate solution, or any other commonly used intravenous solution compatible with the active ingredients. Similarly, such vehicles or acceptable variations thereof could be adapted as needed for intramuscular or intraperitoneal administration. In addition, TX-C or TX-C/ascorbate can be administered in cardioplegic solutions, e.g. Roe's solution, for myocardial protection during cardiovascular surgery. Other compositions to which Trolox C may be added to form composition according to the present invention include isotonic dextrose, ringer's Bretscheider's cardioplegic solution. St Thomas numbers 1,2, and 3, Stanford modified Collins, university of Wisconsin (Belzer's), Kreb s Hensleit and other cardio plegic solutions such as those containing ditliazem, verapamil, nifidepine or a calcium blocker. Moreover, any pharmaceutically accetable oral vehicle compatible with TX-C could be used for oral administration, for example, such gels or capsules as are used for oral administration of Vitamin E.

While it is preferred to use ascorbic acid as an enhancer for TX-C in the compositions of the present invention, effective amounts of other pharmaceutically acceptable materials that are compatible with, and would prevent the oxidation of, TX-C could be substituted for all or part of the ascorbic acid. These may include, for example, mercaptoethanol, dithiothreitol, glutathione, cysteine, and the like. When intended for parenteral use, for example intravenously or intraarterially, it is important that the compositions used are sterile.

The presently preferred process for preparing the presently preferred compositions according to the present invention comprises the steps of:

(1) bubbling nitrogen through physiological saline solution for a period of from about 1 to about 3 hours in order to reduce the oxygen content of said solution;

(2) adding about 30 to about 40 mg of EDTA per liter of the saline solution while continuing nitrogen bubbling;

(3) adding approximately 4 to 6 grams of ascorbic acid per liter of the saline solution while continuing nitrogen bubbling;

(4) adding about 4 grams to about 6 grams of TX-C per liter of the saline solution while stirring and bubbling nitrogen;

(5) slowly adding sodium hydroxide to adjust the pH of the resulting solution to about 12 so as to enhance the solubility of the TX-C;

(6) for solutions for myocardial salvage adding from about 1 to about 4, preferably about 2 grams, of ascorbic acid per liter of the saline solution, for solutions for hepatic salvage adding preferably hydrochloric acid; in either case adding sufficient acid so as to achieve a pH less than or equal to about 6.5 to about 8.0, preferably about 7.2 to about 7.8, most preferably about 7.4; and

(7) slowly adding sodium hydroxide if and to the extent needed to adjust the pH to about 6.5 to about 8.0, preferably about 7.2 to about 7.8, most preferably about 7.4.

In accordance with the presently preferred treatment of the invention, a mammal is protected from reperfusion injury by intravenous or intraarterially administering to the mammal, substantially concurrently with the beginning of reperfusion of tissue to which the flow of blood has been temporarily reduced, as occurs, for example, during a heart attack, coronary surgery, stroke or tissue or organ transplant, a preferred composition of the present invention, the contents and preparation of which have been described above. More preferably, the administration of the composition of the present invention is begun from about 0.5 to about 5 minutes before reperfusion begins, most preferably from about 0.5 to about 1 minute before reperfusion begins and is continued for from about 2 to about 5 minutes after reperfusion has been initiated.

While introduction of the compositions of the present invention directly into the bloodstream is the presently preferred route of administration, the present invention also contemplates intramuscular as well as intraperitoneal administration under appropriate circumstances. Other suitable forms of administration within the scope of the present invention include oral and cardioplegic, either alone or in combination with other forms of administration, such as those described above, particularly for such conditions as scheduled surgery involving heart by-pass or organ or tissue transplant.

The following examples are presented to illustrate the practice of the present invention.

<u>Example 1:</u>

A solution of TX-C was prepared as follows:

500 ml of physiological saline solution was treated by bubbling nitrogen ($N_2$) through it for 2 - 3 hours to decrease the $O_2$ in solution. Then while stirring and continuing to bubble $N_2$, 18 mg of EDTA (ethylenediaminetetraacetic acid) was added, followed by 2000 mg of ascorbic acid. Then 2000 mg of TX-C was added under stirring and $N_2$ bubbling, and the pH was adjusted with NaOH to about 12 to solubilize the TX-C. A spectrally clear (homogeneous) solution was obtained within five minutes. After the TX-C was dissolved, 1000 mg ascorbic acid was added, followed by sufficient NaOH to arrive at a pH of approximately 7.4.

A canine model of 2 hours to LAD (left anterior descending artery) regional ischemia followed by 4 hours of reperfusion was used. The area at risk and the area of infarction were estimated by a dual staining technique using Evan's blue and triphenyl tetrazolium HCl infused at 100mm Hg pressure (physiological coronary perfusion pressure) after cardiac excision. More particularly, in dogs weighing between about 15 and 25 kg, the LAD was occluded for 2 hours and then reperfused for 4 hours. These were the "untreated" controls. In the same model, 500 ml of saline containing 2.0g TX-C, 3.0g ascorbic acid and 18 mg EDTA (prepared as described above) were rapidly infused into the test dogs' ascending aorta beginning 30 sec before reperfusion and then for the initial 3 min of reperfusion. After 4 hours of reperfusion, the hearts were excised and Evans blue dye and tetrazolium staining were employed to identify the areas of risk and the areas infarcted.

As shown in Table 1, medical reperfusion (control) resulted in 27.4 +/-4.8% infarction (N=7) and the Trolox-ascorbate reperfusion produced 8.2 +/-1.6% (N=7) infarction of the area of risk. The unpaired T-test gives a value of 3.82 with 12 degrees of freedom and a $P<0.001$. (To our knowledge this is the most significant decrease in regional myocardial necrosis reported to date after 2 hours of ischemia by any therapeutic intervention.)

The details of the surgical and pathology techniques employed are as follows:

In each case, a mongrel dog is anesthetized with sodium pentobarbitol (30 mg/kg IV), intubated and ventilated with a Harvard respirator. A thoracotomy is performed under sterile conditions through the fifth left intercostal space, the left lung is gently retracted and the pericardium excised. A catheter is placed in the right femoral artery for obtaining blood gas samples. A second catheter is advanced through the left femoral artery into the central aorta for monitoring blood pressure. A third catheter is placed in the femoral vein and used for intravenous injections. The LAD is isolated just distal to the first diagonal and a 3 to 5 mm segment dissected. The heart is carefully inspected and any large collateral vessels from the right or circumflex artery are ligated near the ventricular apex to provide a reproducible region of ischemia. A doppler flow probe is placed around the LAD coronary artery and used to confirm the absence of blood flow with ischemia and the restoration of blood flow with reperfusion.

The area at risk and the area of infarction are estimated by a dual staining technique using Evans Blue and triphenyl tetrazolium hydrochloride, respectively, which are infused at 100mm Hg pressure (physiological coronary perfusion pressure) after cardiac excision. Cannulas are inserted into the proximal aorta and into the LAD at the site of occlusion. The LAD bed is perfused with 1.5% tetrazolium in a 20 mmol phosphate buffer (pH 7.4, 38° C) and the aorta is perfused in an antegrade manner with 0.25% Evans Blue dye.

Table 1

| Control Animals: | | | TX-C Animals: |
|---|---|---|---|
| 20.0 AN/AR* | | | 6.8 AN/AR* |
| 21.6 | | | 5.6 |
| 18.5 | | | 15.9 |
| 20.8 | | | 5.5 |
| 43.6 | | | 8.2 |
| 19.3 | | | 4.1 |
| 47.8 | | | 11.4 |
| 27.4% | | mean | 8.2% |
| 4.8% | | 1 SEM | 1.6% |
| 7 | N | 7 | |
| T test = 3.82 with 12 degrees of freedom. P<0.001 | | | |

* Area of Necrosis (infarction)/Area at risk.

Example 2

A solution of TX-C was prepared as follows:

Nitrogen was bubbled into 500 mL of physiological saline containing 20 mg of EDTA. After 1 hour 3g of ascorbic acid was added, followed by 3g of TX-C. The pH was adjusted to 12 by the addition of 5N NaOH to aid in dissolution of the TX-C. When the TX-C was completely dissolved, the pH was readjusted to 7.4 with 2N hydrochloric acid. During the preparation of the solution, nitrogen was bubbled through the solution continuously.

A rat model of mechanically induced hepatic ischemia was used. The percent necrosis by weight was determined by the method of Federiks et al. [Exp. Mol. Pathol. 41, 119-125(1984)] with the substitution of tetrazolium blue chloride for tetranitro BT staining. Serum AST (aspartate amino transferase) was determined using a Hitachi 705 Autoanalyzer.

Male Sprague Dawley Rats weighing between 300 and 600g were used. Immediately before surgery, the animals were given 10mg gentamycin intramuscularly, anesthetized with an ethrane inhalant, and injected intraperitoneally with heparin(100 U/kg). Next the hepatic artery and portal vein were occluded for 70 minutes using pediatric Satinsky vascular clamps. Beginning 45 seconds prior to release of the occlusion, a 3mL bolus of the Trolox and ascorbic acid solution or a control solution (the normal saline and EDTA vehicle without ascorbic acid or Trolox) were infused for the initial two minutes of reperfusion through the right renal vein using a butterfly needed. The animal was then given a 4ml bolus of 3.75% sodium bicarbonate via the penile vein. Blood was sampled at 0, 12 and 48 hours for the biochemical measurements of aspartate amino transferase (AST) and for conjugated dienes. At the end of this period, the animal was anesthetized and sacrificed by exsanguination. The liver was harvested and placed in saline at room temperature prior to histological analysis. Only those animals that survived to 48 hours were utilized for the analysis of necrosis and serum AST Levels.

It was noted that in 40-50% of the control animals, death resulted within hours after removal of the occlusion; there were no deaths among the TX-C treated animals within the 48 hour test period. The effect on mortality rate is perhaps the most dramatic illustration of the effect of Trolox C in protecting hepatic tissue from reperfusion injury. By utilizing twice as many controls as treated animals roughly equal numbers of livers could be obtained at 48 hours. The control group (N=6)showed 24.5+/-11.0% necrosis whereas the group treated with Trolox (N=7) showed 4.5 +/-2.5% necrosis. Thus 82% of the tissue normally lost on reperfusion after surgically induced liver ischemia was salvaged by the administration of Trolox C. Reflecting the degree of necrosis at 48 hours, the serum AST of the controls was three times that of the Trolox-treated animals: 978 +/- 369 IU/L vs 301 +/- 114 IU/L.

## Claims

1. The use of 6-hydroxy-2,5,7,8-tetramethylchroman-2-carboxylic acid for the manufacture of a medicament for protecting a mammal from tissue injury that is associated with reperfusion following ischemia.

2. A use according to claim 1, wherein said medicament is one suitable for administration directly into the bloodstream.

3. A use according to claim 1, wherein said medicament is one suitable for administration intramuscularly.

4. A use according to claim 1, wherein said medicament is one suitable for administration intraperitoneally.

5. A use according to claim 1, wherein said medicament is suitable for administration orally.

6. A composition comprising 6-hydroxy-2,5,7,8 tetramethylchroman-2-carboxylic acid and ascorbic acid for protecting a mammal from tissue injury associated with reperfusion following ischemia.

7. A protein-free composition for protecting a mammal from tissue injury associated with reperfusion after ischemia, said composition comprising an effective amount of 6-hydroxy-2,5,7,8-tetramethylchroman-2-carboxylic acid and a pharmaceutically acceptable chelating agent in an amount sufficient to chelate any iron present in a pharmaceutically acceptable vehicle.

8. The composition according to claim 7, which further comprises ascorbic acid.

9. The composition of claim 8, which comprises a saline solution comprising water, sodium chloride, said chroman compound, ascorbic acid and ethylenediaminetetraacetic acid.

10. The composition of claim 9, which has a pH in the range of from 6.5 to 8.0, preferably from 7.2 to 7.8, more preferably is about 7.4.

11. The composition of claim 10, which comprises physiological saline solution having added thereto about 3 to 6 preferably 3.5 to 4.5 grams per liter of said chroman compound, from about 4 to about 10 preferably 6 to 8 grams per liter of ascorbic acid, from about 25 to about 50 preferably 30 to 40 mg. per liter of ethylenediaminetetraacetic acid and such amount of sodium hydroxide as is needed to provide said pH.

12. An aqueous composition according to any one of claims 7-11, that is sterile and suitable for parenteral administration.

13. A process for preparing the composition of claim 11, comprising the steps of

(1) bubbling nitrogen through physiological saline solution for a period of from about 1 to about 3 hours in order to reduce the oxygen content of said solution;

(2) adding said ethylenediaminetetraacetic acid while continuing said nitrogen bubbling;

(3) adding approximately 4 to 6g of ascorbic acid per liter of said solution while continuing said nitrogen bubbling;

(4) adding said chroman compound while stirring and bubbling nitrogen;

(5) slowly adding sodium hydroxide to adjust the pH of the resulting solution to about 12 so as to enhance the solubility of said chroman compound;

(6) adding ascorbic acid or hydrochloric acid so as to achieve a pH less than or equal to a value within the range of about 6.5 to about 8.0; and

(7) slowly adding sodium hydroxide if and to the extent needed to adjust the pH to a value within said range.